# EUROPEAN PATENT APPLICATION

(11) **EP 4 563 011 A1**
(43) Date of publication of application: **04.06.2025**
(21) Application number: 24826314.7
(22) Date of filing: 21.06.2024
(51) Int. Cl.: A23L 33/105

(54) **COMPOSITION FOR ENHANCING IMMUNITY**

(30) Priority: 21.06.2023 KR 20230079845
(71) Applicant: Kolmar BNH Co., Ltd, Sejong 30003 (KR)
(72) Inventor: BAK, Su Bin, Seoul 06800 (KR); JU, Jae-Yeong, Seoul 06800 (KR)
(74) Representative: Regimbeau
(86) International application number: PCT/KR2024/008640
(87) International publication number: WO 2024/262999

(57) **Abstract**

The present invention relates to a composition for enhancing immunity, which contains a mixture of a mixed extract of angelica, cnidium, and paeonia and a polysaccharide isolated therefrom; or a fraction thereof as an effective ingredient.

The composition containing a mixture of a mixed extract of angelica, cnidium, and paeonia and a polysaccharide isolated therefrom; or a fraction thereof as an effective ingredient according to the present invention increases body weight, increases WBC count, restores spleen cell density, increases spleen cell proliferation ability, and increases immune-related cytokines as well as significantly increases NK cell activity, particularly with respect to innate immunity, and can be thus applied as an immune-enhancing agent or a food composition for enhancing immunity.

## Description

### [Technical Field]

The present invention relates to a composition for enhancing immunity, which contains a mixture of a mixed extract of angelica, cnidium, and paeonia and a polysaccharide isolated therefrom; or a fraction thereof as an effective ingredient.

### [Background Art]

Immunity is an important biological defense system that protects the body against diseases caused by external pathogens, and the COVID-19 pandemic has highlighted the importance of the functionality and efficiency of immune responses in combating viral infections.

The immune system can be divided into natural resistance, the nonspecific immune system, and the specific immune system. Natural resistance (first line of defense) refers to the anatomical and physiological factors that block all invaders, including microorganisms, regardless of their type. The non-specific immune system (second line of defense) refers to a defense system composed of phagocytes that eliminate invaders that have entered the body by breaking through the natural resistance. The specific immune system (the third line of defense) refers to the immune system composed of lymphocytes. Among these, the specific immune system is a highly developed immune system with memory and the ability to distinguish self from non-self. White blood cells form the second or third line of defense and are responsible for foreign substances that have broken through the first line of defense and entered the body, and during bacterial and viral infections or inflammatory responses, regulation of macrophage and lymphocyte activity plays a pivotal role in determining the therapeutic efficacy of medicine and medical supplies. Immune function regulation is classified into immune function enhancement and immune function suppression. Immune function enhancement means contributing to the enhancement of immune function of the weakened human body, and this also includes cases where normal immune function is enhanced. Immune function suppression is the function of suppressing excessive immune responses. It is typically known that cytokines and nitric oxide, which are immune factors that macrophages overproduce in response to stimulation from external harmful factors such as LPS, cause chronic inflammation and, as a result, cause chronic inflammation-related diseases. However, it is known that an increase in immune factors produced by normal macrophages due to natural substances enhances immunity and suppresses diseases related to immunosuppression.

Immunity can be broadly classified into two types: innate immunity, which individuals are born with, and acquired immunity, which individuals acquire through adaptation to life.

Innate immunity, also called natural immunity, is characterized by a non-specific reaction to antigens and no special memory behavior. **The** innate immune system includes the skin and mucous tissues that block the invasion of antigens, the acidic stomach acid, and complement present in the blood as well as cells such as macrophages and polymorphonuclear leukocytes, which are responsible for phagocytosis, and NK cells, which can kill infected cells. In fact, most infections are protected against by this innate immunity. Acquired immunity, also called adaptive immunity, can remember an antigen that first invaded and respond specifically to the antigen when the antigen invades again to effectively eliminate the antigen, and plays a role in reinforcing innate immunity.

Recently, the field of innate immunity has been receiving attention since the activation of innate immunity can be a fundamental preventive method against various infectious pathogens. When immunity is strong, an individual does not get sick easily, and even if an individual does get sick, the individual recovers quickly and is able to maintain a healthy state. Therefore, managing immunity is greatly important to maintain health and extend lifespan.

However, looking at the lifestyles of modern people, their immunity can easily be weakened by stress, lack of exercise, irregular daily rhythm, unbalanced diet, smoking, and the like, and there are a number of factors that can weaken immunity due to the prevalence of various types of pollution in the living environment as well. Therefore, modern people must make efforts to strengthen their immunity. Hence, studies on natural products to enhance immunity (for example, Korean Patent No. 10-2009-0126681) have been conducted, but studies on angelica, cnidium, and paeonia have not been sufficiently conducted. In particular, the innate immunity strengthening effect of a mixed extract of angelica, cnidium, and paeonia has not been studied.

Accordingly, the present inventors have found that a mixture of a mixed extract of angelica, cnidium, and paeonia and a polysaccharide isolated therefrom; or a fraction thereof increases body weight, increases WBC count, restores spleen cell density, increases spleen cell proliferation ability, and increases immune-related cytokines as well as significantly increases NK cell activity particularly with respect to innate immunity, and can be thus used in a food composition for enhancing immunity, and thus completed the present invention.

### [Disclosure]

### [Technical Problem]

An object of the present invention is to provide a composition for enhancing immunity, which contains a mixture of a mixed extract of angelica, cnidium, and paeonia and a polysaccharide isolated therefrom; or a fraction thereof as an effective ingredient.

### [Technical Solution]

In order to solve the above problem,
an aspect of the present invention is a composition for enhancing immunity, which contains a mixture of a mixed extract of angelica, cnidium, and paeonia and a polysaccharide isolated therefrom; or a fraction thereof as an effective ingredient.

As a specific example, the immunity is innate immunity, acquired immunity or a combination thereof.

As another specific example, the mixed extract is extracted with a solvent selected from the group consisting of water, a linear or branched alcohol having 1 to 6 carbon atoms, an organic solvent, and a mixed solvent of these solvents.

As still another specific example, the mixed extract contains angelica, cnidium, and paeonia at a weight ratio of 1 : 1.2 to 3 : 1.2 to 3.

As still another specific example, the mixed extract is a mixed extract of *Angelica sinensis* (Oliv.) Diels, *Ligusticum chuanxiong* Hort. or *Cnidium officinale,* and *Paeonia lactiflora* Pallas.

As still another specific example, the isolated polysaccharide is an ethanol polysaccharide.

As still another specific example, the ethanol is 95% ethanol.

As still another specific example, the fraction is a water or ethanol fraction of the mixture.

As still another specific example, the water fraction or ethanol fraction has T cell proliferation ability.

As still another specific example, the water fraction or ethanol fraction has a T cell proliferation ability of 130% to 160% at a concentration of 400 to 500 µg/ml.

As still another specific example, the mixture contains a mixed extract of angelica, cnidium, and paeonia and a polysaccharide isolated from the mixed extract at a ratio of 60 to 70 : 30 to 40.

As still another specific example, the mixture is contained at 0.001% to 90% by weight based on a total weight of the composition.

As still another specific example, the mixture or a fraction of the mixture increases NK cell activity.

As still another specific example, the mixture or a fraction of the mixture increases body weight, increases WBC count, restores spleen cell density, increases spleen cell proliferation ability, and increases immune-related cytokines.

As still another specific example, the cytokine is any one or more selected from the group consisting of IL-2, IFN-γ, IL-4, IL-10, TNF-α, IL-12 and IL-6.

As still another specific example, the composition is a food composition.

As still another specific example, the food composition is prepared as a powder, granule, tablet, capsule, syrup or beverage.

Another aspect of the present invention is a use of a composition containing a mixture of a mixed extract of angelica, cnidium, and paeonia and a polysaccharide isolated therefrom; or a fraction thereof as an effective ingredient for enhancing immunity.

Still another aspect of the present invention is a method for enhancing immunity, which includes treating or administering a composition containing a mixture of a mixed extract of angelica, cnidium, and paeonia and a polysaccharide isolated therefrom; or a fraction thereof as an effective ingredient.

### [Advantageous Effects]

The composition containing a mixture of a mixed extract of angelica, cnidium, and paeonia and a polysaccharide isolated therefrom; or a fraction thereof as an effective ingredient according to the present invention increases body weight, increases WBC count, restores spleen cell density, increases spleen cell proliferation ability, and increases immune-related cytokines as well as significantly increases NK cell activity particularly with respect to innate immunity, and can be thus applied as an immune-enhancing agent or a food composition for enhancing immunity.

### [Brief Description of Drawings]

FIG. 1 illustrates the spleen cell proliferation ability of a mixture according to the Preparation Example of the present invention *in vitro*;
FIG. 2 illustrates the expression level of spleen cell cytokines *in vitro* by a mixture according to the Preparation Example of the present invention;
FIG. 3 illustrates the T cell proliferation ability of a fraction of a mixture according to the Preparation Example of the present invention *in vitro*;
FIG. 4 illustrates a schematic diagram showing the overall experimental period for the induction of immunosuppression when an animal model according to the Preparation Example of the present invention is constructed and the administration of a mixture (HG) according to Example of the present invention;
FIG. 5 illustrates weight loss and immunosuppression induction after CTX administration according to the Preparation Example of the present invention and the *in vivo* body weight recovery ability when a mixture (HG) according to the Example of the present invention is administered;
FIG. 6 illustrates immunosuppression induction after CTX administration according to the Preparation Example of the present invention and the *in vivo* WBC recovery ability when a mixture (HG) according to the Preparation Example of the present invention is administered;
FIG. 7 illustrates an increase in *in vivo* NK cell activity when a mixture (HG) according to the Preparation Example of the present invention is administered;
FIG. 8 illustrates recovery of the *in vivo* spleen cell density when a mixture (HG) according to the Preparation Example of the present invention is administered;
FIG. 9 illustrates an increase in the expression of *in vivo* immune-related cytokines when a mixture (HG) according to the Preparation Example of the present invention is administered; and
FIG. 10 illustrates *in vivo* spleen cell proliferation ability when a mixture (HG) according to the Preparation Example of the present invention is administered.

### [Detailed Description of the Invention]

Each description and each embodiment disclosed in this application may also be applied to another description and another embodiment, respectively. In other words, all combinations of the various elements disclosed in this application fall within the scope of the present invention. Additionally, the scope of the present invention is not limited by the specific description described below.

Furthermore, those skilled in the art will recognize or be able to ascertain, using no more than routine experimentation, many equivalents to the specific aspects of the present invention described in this application. Additionally, such equivalents are intended to be included in the present invention.

In addition, throughout the specification of this application, when a part is said to "include" a certain component, this does not mean that other components are excluded, but rather that other components may be further included, unless otherwise specifically stated.

Hereinafter, the present invention will be described in more detail.

The present invention is based on the discovery that a mixture of a mixed extract of angelica, cnidium, and paeonia and a polysaccharide isolated therefrom; or a fraction thereof increases body weight, increases WBC count, restores spleen cell density, increases spleen cell proliferation ability, and increases immune-related cytokines as well as significantly increases NK cell activity, particularly with respect to innate immunity, and thus enhances immunity, and can be utilized as an immune-enhancing agent or immune tonic.

An aspect of the present invention to achieve the above object provides a composition for enhancing immunity, which contains a mixture of a mixed extract of angelica, cnidium, and paeonia and a polysaccharide isolated therefrom; or a fraction thereof as an effective ingredient.

In the present invention, the term "angelica (dong quai)" means the dried root of *Angelica sinensis* (Oliv.) Diels, and this is distinct from the roots of *Angelica gigas* Nakai in Korea and *Angelica acutiloba* (Siebold. & Zucc.) Kitag. or *Angelica acutiloba* (Siebold. & Zucc.) Kitag. var. sugiyamae Hikino in Japan. *Angelica sinensis* is warm in energy and has a sweet and spicy taste. In general, *Angelica sinensis* is sweeter and less spicy than *Angelica gigas*. The efficacy of *Angelica sinensis* is to replenish blood when there is a lack of blood, and dong quai, made of the root of the *Angelica sinensis,* has an excellent blood-replenishing effect. However, dong quai, made of the root of *Angelica gigas*, is more effective in promoting blood circulation than in replenishing blood, and has strong anticancer and blood pressure-lowering effects. Pharmacologically, it is known that dong quai promotes blood flow in coronary arteries and stimulates red blood cell production.

In the present invention, the term "cnidium" refers to *Ligusticum chuanxiong* Hort. or *Cnidium officinale,* which is a perennial herb of the *Apiaceae* family, Apiaceae order, and *Dicotyledonous* group of dicotyledons, and is commonly cultivated as a medicinal plant. Cnidium is effective for sedation, pain relief, and strengthening, and is used to treat headaches, anemia, and gynecological diseases. The rhizome of cnidium is dug up in September to November, the leaves and stems are removed, and the rhizome is dried in the sun and then boiled and taken or made into pills or powders for use.

In the present invention, the term "paeonia (*Paeonia lactiflora* Pallas)" refers to a perennial herb of the *Paeonia* genus in the *Paeoniaceae* family, a dicotyledonous plant growing in mountainous areas, and is used for horticultural purposes because of its beautiful flowers. Additionally, the root is used as a medicinal herb for pain, abdominal pain, menstrual cramps, amenorrhea, vomiting blood, anemia, and bruises. Paeonia has been cultivated as an ornamental plant in China since the Qin and Ming Dynasties, and its cultivation history is longer than that of *Paeonia suffruticosa* Andr. Dozens of varieties of Paeonia were recorded during the Song and Qing Dynasties, and are distributed in Korea, Mongolia, and East Siberia.

In the present specification, paeonia may include one or more selected from the group consisting of *Paeonia lactiflora* Pallas, *Paeonia obovata var. japonica*, *Paeonia* sect. *Paeonia*, *Paeonia lactiflora for. pilosella Nakai*, and *Paeonia lactiflora var. trichocarpa (Bunge) Stern*, and can be used without limitation regardless of the processing method.

The above-mentioned angelica, cnidium, and paeonia can be purchased commercially, or those collected from nature or cultivated ones may be used without limitation.

In the present invention, the term "extract" refers to a mixed extract containing the above-mentioned angelica, cnidium, and paeonia, and the mixed extract of angelica, cnidium, and paeonia may be obtained from various organs of natural, hybrid, and mutant plants, and specifically, may be extracted from roots, aerial parts, stems, leaves, flowers, fruit bodies, and fruit peels as well as plant tissue cultures.

The "mixed extract" is a mixed extract containing all of angelica, cnidium, and paeonia, and may be an extract obtained by first mixing the three substances and then subjecting the mixture to extraction, or may be an extract obtained by subjecting each substance to extraction individually and then mixing the extracts thus prepared, and includes, without limitation, any mixed extract prepared by a method capable of preparing a mixed extract by any other known method.

The extract can be obtained by subjecting the pulverized product of each component to extraction with a solvent selected from the group consisting of water, a linear or branched alcohol having 1 to 6 carbon atoms, an organic solvent, and a mixed solvent thereof, but is not limited thereto. The extract may be an extract obtained by performing extraction in an extraction temperature range of 20°C to 100°C for an extraction period of about 1 hour to 10 days by an extraction method such as hot water extraction, cold extraction, reflux cooling extraction, or ultrasonic extraction, but the extraction is not limited to the above-mentioned extraction temperature, extraction period or extraction method.

In a case where the solvent of the extract is water, the extract may include a cold-water extract or a hot water extract.

The organic solvent is not particularly limited and may be polyhydric alcohols such as glycerol, ethylene glycol, propylene glycol, and 1,3-butylene glycol; hydrocarbon-based solvents such as methyl acetate, ethyl acetate, benzene, n-hexane, diethyl ether, dichloromethane, and chloroform; and nonpolar organic solvents such as petroleum ether, methyl acetate, benzene, hexane, chloroform, methylene chloride, dimethyl ether, and ethyl acetate.

The solvent may also include an aqueous solution of an organic solvent, and the concentration of which is not particularly limited, but may be 1% to 99% (v/v), specifically 60% to 98% (v/v), more specifically 80% to 95% (v/v), still more specifically 95% (v/v).

The extract may include all of an extraction liquid, a diluted or concentrated extraction liquid, a dried product obtained by drying an extraction liquid, and modified or purified products of these.

The extract may contain angelica, cnidium, and paeonia at a weight ratio of 1 to 10 : 1 to 10 : 1 to 10, specifically a weight ratio of 1 to 5 : 1 to 5 : 1 to 5, more specifically a weight ratio of 1 : 1.2 to 3 : 1.2 to 3, still more specifically a weight ratio of 1 : 1.3 to 2 : 1.2 to 2, still more specifically a weight ratio of 1 : 1.4 to 1.7 : 1.2 to 1.6, still more specifically a weight ratio of 1 : 1.5 to 1.7 : 1.2 to 1.5, still more specifically a weight ratio of 1 : 1.6 : 1.3.

As a specific example, the extract may be a mixed extract containing *Angelica sinensis* (Oliv.) Diels, *Ligusticum chuanxiong* Hort. or *Cnidium officinale,* and *Paeonia lactiflora* Pallas at a weight ratio of 1 : 1.6 : 1.3.

As a specific example, the extract may be a mixed extract containing *Angelica sinensis* (Oliv.) Diels, *Ligusticum chuanxiong* Hort. and *Paeonia lactiflora* Pallas at a weight ratio of 1 : 1.6 : 1.3.

To the extract of the present invention, an extract of a natural product or a known component that enhances or strengthens immunity may optionally be added.

In the present invention, the term "isolated polysaccharide" includes a polysaccharide precipitated by adding a solvent to the mixed extract of angelica, cnidium, and paeonia. Here, the polysaccharide is also called a polysaccharide and may contain one or more polysaccharides.

In the present invention, the method for obtaining the isolated polysaccharide is not particularly limited, and the isolated polysaccharide may be obtained according to a method commonly used in the art. As non-limiting examples of the method, the isolated polysaccharide may be an isolated polysaccharide obtained from the extracts of angelica, cnidium, and paeonia of the present invention by treating the extracts with a predetermined solvent, and specifically may be an isolated polysaccharide produced by adding an organic solvent to the extracts of angelica, cnidium, and paeonia of the present invention, but is not limited thereto.

In the present invention, the type of solvent used to obtain the isolated polysaccharide is not particularly limited, and an arbitrary solvent known in the art may be used. Non-limiting examples of the solvent include water, a linear or branched alcohol having 1 to 6 carbon atoms, an organic solvent, and a mixed solvent thereof, and these may be used singly or in a mixture of one or more, but the solvent is not limited thereto. The organic solvent may be one or more selected from the group consisting of dichloromethane, diethyl ether, chloroform, and ethyl acetate, but is not limited thereto.

Specifically, the isolated polysaccharide may be an ethanol polysaccharide, more specifically a 95% ethanol polysaccharide.

In the present invention, the term "mixture" means a mixture in which the extracts of angelica, cnidium, and paeonia prepared in the present invention are mixed with the isolated polysaccharides obtained by treating each of the extracts with a predetermined solvent, and the mixing ratio thereof is not particularly limited.

As the composition contains extracts of the respective plants and polysaccharides isolated therefrom in combination, remarkably superior effects can be obtained compared to cases where only an extract of one plant and a polysaccharide isolated therefrom or polysaccharide thereof are contained. The composition can exhibit a remarkably superior immunity enhancing or strengthening effect compared to a case where the composition contains extracts of plants other than angelica, cnidium, and paeonia, a polysaccharide isolated therefrom, or a polysaccharide thereof.

When the mixture is a mixture of an extract and a polysaccharide isolated therefrom, the extract and the polysaccharide isolated therefrom may be contained at a weight ratio of about 40 to 80 : 20 to 60, specifically at a weight ratio of 45 to 75 : 25 to 55, more specifically at a weight ratio of 50 to 70 : 30 to 50, still more specifically at a weight ratio of 60 to 70 : 30 to 40, still more specifically at a weight ratio of 60 : 40, but are not limited thereto.

In the present invention, the term "fraction thereof" means a product obtained by performing fractionation to isolate a specific component or a specific component group from a mixture containing various components.

In the present invention, the fractionation method to obtain the fraction is not particularly limited, and the fraction may be obtained according to a method commonly used in the art. As non-limiting examples of the fractionation method, the fraction may be a fraction obtained by treating a mixture in which extracts of angelica, cnidium, and paeonia of the present invention and polysaccharides thereof are mixed with a predetermined solvent.

In the present invention, the type of fractionation solvent used to obtain the fraction is not particularly limited, and an arbitrary solvent known in the art may be used. Non-limiting examples of the fractionation solvent include water, a linear or branched alcohol having 1 to 6 carbon atoms, an organic solvent, and a mixed solvent thereof, and these may be used singly or in a mixture of one or more, but the solvent is not limited thereto. The organic solvent may be one or more selected from the group consisting of dichloromethane, diethyl ether, chloroform, and ethyl acetate, but is not limited thereto.

The fraction may be specifically a fraction produced by adding water or ethanol to a mixture in which extracts of angelica, cnidium, and paeonia of the present invention and polysaccharides isolated therefrom are mixed, more specifically a water fraction or ethanol fraction of a mixture in which extracts of angelica, cnidium, and paeonia and polysaccharides isolated therefrom are mixed.

More specifically, the fraction may be a water fraction of a mixture in which extracts of angelica, cnidium, and paeonia and polysaccharides isolated therefrom are mixed.

The water fraction or ethanol fraction of the mixture of the present invention may be characterized by having T cell proliferation ability, and the water fraction or ethanol fraction of the mixture of the present invention may be characterized by having a T cell proliferation ability of specifically 130% to 160%, more specifically 135% to 155% at a concentration of 400 to 500 µg/ml.

The mixture of extracts of angelica, cnidium, and paeonia and polysaccharides isolated therefrom, or a fraction thereof may be contained at 0.0001% to 90% by weight based on the total weight of the composition, and the mixture or a fraction thereof may be contained at specifically 30% to 80% by weight, more specifically 35% to 70% by weight, still more specifically 40% to 65% by weight based on the total weight of the composition, but is not limited thereto.

The mixture of extracts of angelica, cnidium, and paeonia and polysaccharides isolated therefrom, or a fraction thereof can increase NK cell activity.

The term "NK cell" is known to function in the spleen and bone marrow and regulate innate immunity in the body, and NK cell activity can be measured by IFN-γ expression.

The mixture of extracts of angelica, cnidium, and paeonia and polysaccharides isolated therefrom, or a fraction thereof can increase body weight, increase WBC count, restore spleen cell density, increase spleen cell proliferation ability, and increase immune-related cytokines.

The immune-related cytokines may be any one or more selected from the group consisting of IL-2, IFN-γ, IL-4, IL-10, TNF-α, IL-12, and IL-6.

In the present invention, the term "improvement" means enhancing immunity, strengthening immunity, or reinforcing immunity by applying the mixture of extracts of angelica, cnidium, and paeonia and polysaccharides isolated therefrom, or a fraction thereof to a composition.

The composition, which includes the mixture of extracts of angelica, cnidium, and paeonia and polysaccharides isolated therefrom, or a fraction thereof, may be added to a food composition for the purpose of enhancing, improving, strengthening, or reinforcing immunity, and the food composition may be a health functional food composition.

The food composition may contain a sitologically acceptable carrier.

The food composition of the present invention includes all forms such as functional food, nutritional supplement, health food, and food additives, and the above type of food composition may be prepared in various forms according to conventional methods known in the art.

In a case where the mixture of extracts of angelica, cnidium, and paeonia and polysaccharides isolated therefrom, or a fraction thereof is used as a food additive, the mixture of extracts of angelica, cnidium, and paeonia and polysaccharides isolated therefrom, or a fraction thereof may be added as it is or may be used together with other foods or food ingredients, and may be used appropriately according to conventional methods. The amount of active ingredients mixed may be properly determined depending on the purpose of use (prevention, health, or therapeutic treatment). Generally, when a food or beverage is prepared, the mixture of extracts of angelica, cnidium, and paeonia and polysaccharides isolated therefrom, or a fraction thereof is added to the raw material composition in an amount of 0.0001 % to 90% by weight, preferably 0.001 % to 50% by weight. However, in the case of long-term intake for health and hygiene purposes or health control purposes, the amount may be used below the above range.

The type of food is not particularly limited. Examples of the food to which the substances can be added include meat, sausage, bread, chocolate, candy, snacks, confectionery, pizza, ramen, other noodles, gum, dairy products including ice cream, various soups, beverages, tea, drinks, alcoholic beverages, and vitamin complexes, and may include all health foods in the conventional sense.

The health beverage composition of the present invention may contain various flavoring agents, natural carbohydrates, or the like as additional ingredients, like conventional beverages. The natural carbohydrates mentioned above are monosaccharides such as glucose and fructose, disaccharides such as maltose and sucrose, polysaccharides such as dextrin and cyclodextrin, and sugar alcohols such as xylitol, sorbitol, and erythritol. As sweeteners, natural sweeteners such as thaumatin and stevia extract or synthetic sweeteners such as saccharin and aspartame may be used. The proportion of the natural carbohydrates may be generally about 0.001 to 50 parts by weight, specifically about 0.01 to 30 parts by weight per 100 parts by weight of the composition of the present invention.

In addition to the above, the composition of the present invention may contain various nutrients, vitamins, electrolytes, flavoring agents, coloring agents, pectic acid and its salts, alginic acid and its salts, organic acids, protective colloid thickeners, pH adjusters, stabilizers, preservatives, glycerin, alcohols, carbonating agents used in carbonated beverages, and the like. The proportion of these additives is not greatly important, but is typically selected in the range of 0.01 to 30 parts by weight per 100 parts by weight of the composition of the present invention. In addition, the composition of the present invention may contain fruit pulp for preparation of natural fruit juice, fruit juice beverages, and vegetable beverages. The proportion of the pulp is not greatly important, but is generally selected in the range of 0.01 to 30 parts by weight per 100 parts by weight of the composition of the present invention, and these ingredients may be used independently or in combination.

Hereinafter, the present invention will be described in more detail with reference to Examples. However, these Examples are intended to exemplify the present invention, and the scope of the present invention is not limited by these Examples, as will be apparent to those skilled in the art to which the present invention pertains.

### Example 1. Preparation of composition

### 1-1. Preparation of mixture derived from angelica, cnidium, and paeonia

### 1-1-1. Preparation of mixed extract of angelica, cnidium, and paeonia

The root of angelica, rhizome of cnidium, and root of paeonia were each dried in the shade, cut into pieces, and mixed. Then distilled water amounting to 10 times the total weight of the respective herbal medicines (1,000 ml of distilled water per 100 g of herbal medicines) was added, and hot water extraction was performed at 95°C for 4 hours to obtain an extraction liquid. The extracts were then combined, concentrated under reduced pressure, and filtered to obtain a mixed extract.

At this time, either *Angelica sinensis* (Oliv.) Diels or *Angelica gigas* Nakai was chosen as angelica, either *Ligusticum chuanxiong* Hort. or *Cnidium officinale* was chosen as cnidium, and only *Paeonia lactiflora* Pallas was chosen as paeonia, and these were mixed at various weight ratios to prepare mixed extracts (see Table 1).

### 1-1-2. Preparation of polysaccharide isolated from mixed extract

A polysaccharide isolated from each of the mixed extracts prepared in Example 1-1 was prepared.

Specifically, the mixed extract prepared in Example 1-1 was taken, 95% ethanol was added thereto in an amount equal to four times the volume of the mixed extract, the mixture was left to stand at 25°C or less for 16 hours, and then centrifugation was performed to obtain a precipitated polysaccharide isolated from each mixed extract.

### 1-1-3. Preparation of mixture of mixed extract and isolated polysaccharide

A mixture in which the mixed extract prepared in Example 1-1 and the isolated polysaccharide prepared in Example 1-2 were mixed was prepared.

Specifically, each mixture was prepared by mixing each of the mixed extracts prepared in Example 1-1 and each of the isolated polysaccharides prepared in Example 1-2. Thereafter, each of the prepared mixtures was filtered through sterile filter paper (Millipore membrane, 0.45 µm) to maintain a sterile state.

At this time, the mixed extract of Example 1-1 and the isolated polysaccharide of Example 1-2 were mixed at a weight ratio of 60 : 40.

The mixtures are all summarized as shown in Table 1 below.

**[Table 1]**

| Experimental group* | Angelica | Cnidium | Paeonia | Ratio |
|---|---|---|---|---|
| Preparation Example 1 | *Angelica sinensis* (Oliv.) Diels | *Ligusticum chuanxiong* Hort. | *Paeonia lactiflora* Pallas | 1:1.6:1.3 |
| Preparation Example 2 | *Angelica sinensis* (Oliv.) Diels | *Ligusticum chuanxiong* Hort. | *Paeonia lactiflora* Pallas | 1:1.5:1.2 |
| Preparation Example 3 | *Angelica sinensis* (Oliv.) Diels | *Ligusticum chuanxiong* Hort. | *Paeonia lactiflora* Pallas | 1:3:3 |
| Preparation Example 4 | *Angelica sinensis* (Oliv.) Diels | *Cnidium officinale* | *Paeonia lactiflora* Pallas | 1:1.6:1.3 |
| Preparation Example 5 | *Angelica sinensis* (Oliv.) Diels | *Ligusticum chuanxiong* Hort. | *Paeonia lactiflora* Pallas | 1:1:1 |
| Preparation Example 6 | *Angelica sinensis* (Oliv.) Diels | *Ligusticum chuanxiong* Hort. | *Paeonia lactiflora* Pallas | 1:4:1 |
| Preparation Example 7 | *Angelica sinensis* (Oliv.) Diels | *Ligusticum chuanxiong* Hort. | *Paeonia lactiflora* Pallas | 1:1:4 |
| Preparation Example 8 | *Angelica sinensis* (Oliv.) Diels | *Ligusticum chuanxiong* Hort. | *Paeonia lactiflora* Pallas | 1:3.5:3.5 |
| Preparation Example 9 | *Angelica gigas* Nakai | *Cnidium officinale* | *Paeonia lactiflora* Pallas | 1:1:1 |
| Preparation Example 10 | *Angelica gigas* Nakai | *Cnidium officinale* | *Paeonia lactiflora* Pallas | 1:1.6:1.3 |
| Preparation Example 11 | *Angelica gigas* Nakai | *Ligusticum chuanxiong* Hort. | *Paeonia lactiflora* Pallas | 1:1.6:1.3 |

| | | | | |
|---|---|---|---|---|
| * The respective experimental groups, Preparation Examples 1 to 11, are in a form in which of angelica, cnidium, and paeonia are mixed at the corresponding weight ratio, extraction is performed, a polysaccharide (ethanol polysaccharide) isolated from each extract is prepared, and then the prepared mixed extract and isolated polysaccharide are mixed, and refer to a mixture in which an isolated polysaccharide is mixed although the isolated polysaccharide is not described in Table 1. | | | | |

### 1-2. Preparation of fraction using mixture of mixed extract and isolated polysaccharide

A fraction of a mixture in which a mixed extract of angelica, cnidium, and paeonia and a polysaccharide isolated therefrom were mixed was prepared as shown in Table 2 below.

Specifically, a fraction of the mixture in which a mixed extract of *Angelica sinensis* (Oliv.) Diels, *Ligusticum chuanxiong* Hort., and *Paeonia lactiflora* Pallas and a polysaccharide (ethanol polysaccharide) isolated therefrom was mixed in Preparation Example 1 was prepared using water or ethanol as a solvent.

A fraction of the mixture in which a mixed extract of *Angelica sinensis* (Oliv.) Diels, *Ligusticum chuanxiong* Hort. and *Paeonia lactiflora* Pallas and a polysaccharide (ethanol polysaccharide) isolated therefrom was mixed in Preparation Example 5 was also prepared using the same solvent as above.

The fractions are summarized as shown in Table 2 below.

**[Table 2]**

| Experimental group | Angelica | Cnidium | Paeonia | Ratio | Solvent |
|---|---|---|---|---|---|
| Fraction of Preparation Example 1 | *Angelica sinensis* (Oliv.) Diels | *Ligusticum chuanxiong* Hort. | *Paeonia lactiflora* Pallas | 1:1.6:1.3 | Water/ethanol |
| Fraction of Preparation Example 5 | *Angelica sinensis* (Oliv.) Diels | *Ligusticum chuanxiong* Hort. | *Paeonia lactiflora* Pallas | 1:1:1 | Water/ethanol |

### Example 2. Examination of immunity enhancing effect in vitro

### 2-1. Examination of spleen cell proliferation ability

The spleen cell proliferation ability of the Preparation Example prepared in Example 1 was examined.

Specifically, the mouse spleen was collected, spleen cells were extracted, a 96-well plate was prepared, and the cells were seeded at 1 × 10⁶ cells/ml per well. Afterward, each sample was diluted with a cell medium to have a final concentration of 500 µg/ml, and the cells were treated with the diluted sample and incubated under 37°C and 5% CO₂ conditions for 36 hours. After 36 hours, the cells were treated with WST-8 (cell viability assay kit) and reacted under 37°C and 5% CO₂ conditions for 1 hour in a dark state. After the reaction was completed, the OD value was measured as the absorbance at 450 nm, and the measured OD value was calculated to compare the cell proliferation ability value with that of the control.

At this time, PMA 50 ng/ml and ionomycin 2 µg/ml were used as positive controls.

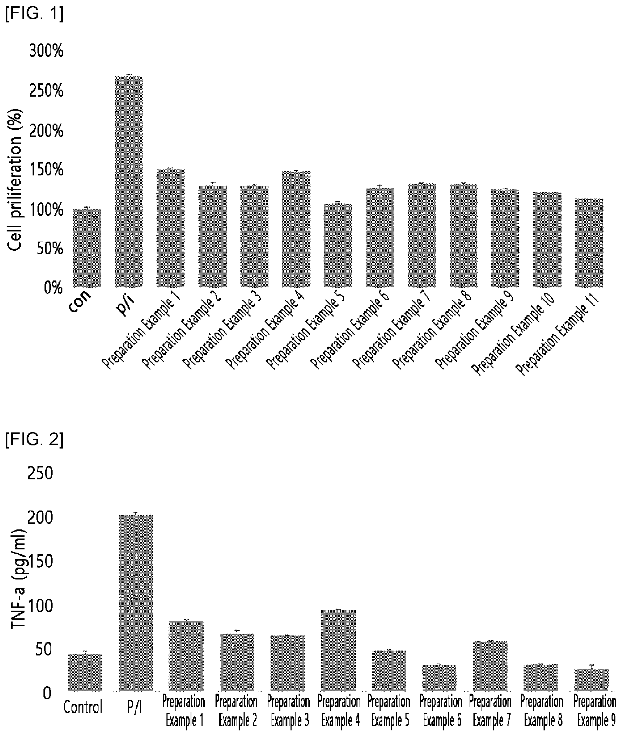

The results of examining the spleen cell proliferation ability of the mixtures are shown in Table 3 and FIG. 1.

**[Table 3]**

| Mean | | | |
|---|---|---|---|
| Con | 100% | p/i | 268% |
| Preparation Example 1 | 150% | Preparation Example 2 | 130% |
| Preparation Example 3 | 130% | Preparation Example 4 | 147% |
| Preparation Example 5 | 108% | Preparation Example 6 | 128% |
| Preparation Example 7 | 132% | Preparation Example 8 | 132% |
| Preparation Example 9 | 125% | Preparation Example 10 | 121% |
| Preparation Example 11 | 113% | | |

As can be seen from FIG. 1 and Table 3, it was found that the mixtures of Preparation Examples 1 to 3 in which *Angelica sinensis* (Oliv.) Diels, *Ligusticum chuanxiong* Hort. and *Paeonia lactiflora* Pallas were mixed and the mixture of Preparation Example 4 in which *Angelica sinensis* (Oliv.) Diels, *Cnidium officinale,* and *Paeonia lactiflora* Pallas were mixed were all superior in spleen cell proliferation ability to the mixtures of Preparation Examples 5 to 11 in which these plants were mixed at mixing ratios different from those in Preparation Examples 1 to 4 or *Angelica sinensis* (Oliv.) Diels was not used.

Among others, in the case of Preparation Examples 1 and 4 in which *Angelica sinensis* (Oliv.) Diels, *Ligusticum chuanxiong* Hort. or *Cnidium officinale,* and *Paeonia lactiflora* Pallas were mixed at a weight ratio of 1 : 1.6 : 1.3, the spleen cell proliferation ability increased to about 150%, and it was found that Preparation Examples 1 and 4 were remarkably superior in spleen cell proliferation ability by a minimum of 20% to a maximum of 50% compared to Preparation Examples 5 to 11 in which the spleen cell proliferation ability increased to about 100% to 130%.

Through this, it can be seen that a mixture prepared using *Angelica sinensis* (Oliv.) Diels, *Ligusticum chuanxiong* Hort. or *Cnidium officinale,* and *Paeonia lactiflora* Pallas has a greatly excellent effect in enhancing immunity.

### 2-2. Examination of expression level of spleen cell cytokine TNF-α

Next, the expression level of spleen cell cytokines by Preparation Examples 1 to 9 prepared in Example 1 was examined. Among cytokines, the expression level of TNF-α was examined as representative.

Specifically, the mouse spleen was collected, spleen cells were extracted, and a 48-well plate was prepared. Afterward, cell solution was prepared at a concentration of 1 × 10⁶ cells/ml and seeded into the 48-well plate. The cells were treated with each sample diluted to have a concentration of 500 µg/ml, and incubated for 48 hours under 37°C and 5% CO₂ conditions. A 96-well maxibinding plate was coated with the target antibody to be examined, and ELISA was performed, and then the antibody expression level was measured as the absorbance at 450 nm. The results were analyzed while comparing the cytokine expression levels due to the treatment with a sample and are shown in Table 4 and FIG. 2.

As a positive control, the cells were treated with PMA 50 ng/ml and ionomycin 2 µg/ml in combination and used.

**[Table 4]**

| Mean | | | |
|---|---|---|---|
| Con | 44.5 | p/i | 202.655 |
| Preparation Example 1 | 81.8355 | Preparation Example 2 | 66.612 |
| Preparation Example 3 | 64.9375 | Preparation Example 4 | 93.4715 |
| Preparation Example 5 | 47.542 | Preparation Example 6 | 31.1455 |
| Preparation Example 7 | 57.987 | Preparation Example 8 | 31.7095 |
| Preparation Example 9 | 26.314 | | |

As a result, as can be seen from Table 4 and FIG. 2, it was found that Preparation Examples 1 and 4, the mixtures prepared using *Angelica sinensis* (Oliv.) Diels, *Ligusticum chuanxiong* Hort. or *Cnidium officinale,* and *Paeonia lactiflora* Pallas, all increased the expression level of TNF-α, a spleen cell cytokine, when compared with Preparation Examples 5 to 9 in which the mixing ratios of these plants were different from those in Preparation Examples 1 and 4.

Through this, it can be seen that a mixture prepared using *Angelica sinensis* (Oliv.) Diels, *Ligusticum chuanxiong* Hort. or *Cnidium officinale,* and *Paeonia lactiflora* Pallas has a greatly excellent effect in enhancing immunity.

### 2-3. Examination of T cell proliferation efficacy of fraction

In order to examine which immune-activating fraction the mixtures prepared in Example 1-1 contained, the immunity enhancing effect of the fractions prepared in Example 1-2 was examined through T cell proliferation ability. The T cell proliferation ability was compared mainly using the fractions of Preparation Examples 1 and 5.

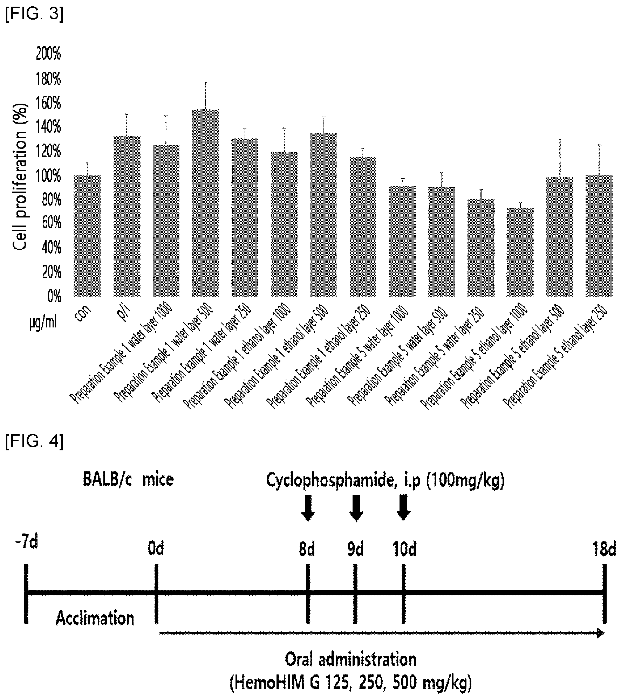

Specifically, the Jurkat cell line was incubated in RPMI-1640 medium supplemented with 10% FBS and 1% antibiotic/antimycotic. The cells were seeded in the prepared 96-well plate at 1 × 10⁶ cells/ml per well. Incubation was performed under 37°C and 5% CO₂ conditions for 36 hours, and then the cells were treated with each sample diluted to have a concentration of 500 µg/ml. After 47 hours, the cells were treated with WST-8 (cell viability assay kit) and reacted under 37°C and 5% CO₂ conditions for 1 hour in a dark state. After the reaction was completed, the OD value was measured as the absorbance at 450 nm, and this was calculated to compare the cell proliferation ability with that of the control, and the results are shown in Table 5 and FIG. 3.

**[Table 5]**

| | | | Preparation Example 1 | | | | | | Preparation Example 5 | | | | | |
|---|---|---|---|---|---|---|---|---|---|---|---|---|---|---|
| | | | Water layer | | | Ethanol layer | | | Water layer | | | Ethanol layer | | |
| | con | p/i | 1000 | 500 | 250 | 1000 | 500 | 250 | 1000 | 500 | 250 | 1000 | 500 | 250 |
| AVE RA GE | 100 % | 132 % | 125% | 154 % | 130 % | 119% | 135 % | 115 % | 91% | 90 % | 80 % | 73% | 99 % | 100 % |

As a result, as can be seen from Table 5 and FIG. 3, it was found that the fraction of Preparation Example 1, a mixture obtained by mixing a mixed extract in which *Angelica sinensis* (Oliv.) Diels, *Ligusticum chuanxiong* Hort. and *Paeonia lactiflora* Pallas were mixed at a weight ratio of 1 : 1.6 : 1.3 with a polysaccharide (ethanol polysaccharide) isolated therefrom, had a significantly superior T cell proliferation ability compared to the fraction of Preparation Example 5 in which the mixing ratio of these plants was different from that in Preparation Example 1. In particular, it can be seen that a water fraction and an ethanol fraction of the mixture of the present invention have a greatly excellent T cell proliferation ability of 154% and 135%, respectively, at a concentration of 400 to 500 µg/ml.

Accordingly, among the fractions of Preparation Example 1, the water fraction and the ethanol fraction have greatly excellent T cell proliferation ability, and it has been found that the water fraction and the ethanol fraction have a particularly excellent immunity enhancing effect.

Furthermore, through the above results, it can be seen that the water fraction and ethanol fraction obtained from the mixture of the present invention exhibit remarkably excellent T cell proliferation ability, and this suggests that the mixture of the present invention, which is the source of the fractions, exhibits excellent immunity enhancing activity.

### Example 3. Examination of immunity enhancing effect in vivo

The mixture (HG) obtained by mixing a mixed extract in which *Angelica sinensis* (Oliv.) Diels, *Ligusticum chuanxiong* Hort., and *Paeonia lactiflora* Pallas were mixed at a weight ratio of 1 : 1.6 : 1.3 with an ethanol polysaccharide thereof from Preparation Example 1, which was found to exhibit the best effect in Experimental Example 2, was chosen and the immunity enhancing effect thereof in *vivo* was examined.

### 3-1. Construction of immunocompromised animal model

As experimental animals, 60 10- to 11-week-old Balb/c mice (male, Dooyeol Biotech, Korea) were prepared, and the test period took approximately 3 weeks.

Specifically, the mice were acclimatized for one week and housed together in cages of up to five mice each, maintaining a temperature of 23°C ± 2°C, a humidity of 60% ± 5%, and a 12-hour light/dark cycle (light/dark cycle from 8:00 AM to 8:00 PM). After one week, each mouse was assigned to one of the treatment groups based on its body weight.

The experimental groups are as shown in Table 6 below.

**[Table 6]**

| Group | n number |
|---|---|
| CTL (Negative control) | 12 |
| CTX (Positive control) | 12 |
| HG125 (mixture prepared as Preparation Example 1 HG 125 mg/kg) | 12 |
| HG250 (mixture of present invention prepared as Preparation Example 1 HG 250 mg/kg) | 12 |
| HG500 (mixture of present invention prepared as Preparation Example 1 HG 500 mg/kg) | 12 |

Next, in order to prepare an immunocompromised animal model, CTX (cyclophosphamide) was administered intraperitoneally at 100 mg/kg, and the specific administration method is as shown in FIG. 4.

Specifically, HG was administered orally to the experimental groups for 7 days starting 7 days before induction of immunosuppression (before CTX administration).

For the next 3 days (from the 8th day to the 10th day), CTX and the mixture HG prepared as Preparation Example 1 were administered simultaneously. Next, the mixture HG of Preparation Example 1 was additionally administered for 8 days (from the 11th day to the 18th day).

Thereafter, using the constructed immunocompromised animal model, the weight gain due to the mixture (HG) prepared as Preparation Example 1 of the present invention, blood cell analysis (WBC), NK cell activity (IFN-γ) analysis, spleen H&E staining analysis, and spleen cell cytokines were sequentially examined. The specific results are as in Examples 3-2 to 3-5 below.

In addition, the spleen cell proliferation ability of Preparation Example 1 was examined using a normal animal model, and the results are as in Example 3-6.

### 3-2. Examination of induction of immunosuppression through CTX administration and examination of body weight and WBC recovery by administration of mixture (HG) prepared as Preparation Example 1

After CTX administration, whether immunosuppression was properly induced was first examined through body weight and CBC analysis. During this time, body weight was measured daily.

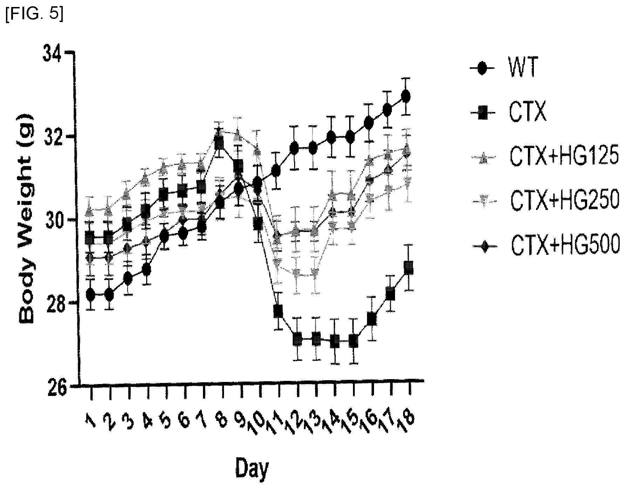

As a result, as can be seen from FIG. 5, it was found that body weight was reduced in all test groups after a total of three administrations of CTX at 100 mg/kg. This suggests that immunosuppression was induced after CTX administration.

Afterwards, the mixture (HG) of a mixed extract of *Angelica sinensis* (Oliv.) Diels, *Ligusticum chuanxiong* Hort. and *Paeonia lactiflora* Pallas and a polysaccharide (ethanol polysaccharide) isolated therefrom prepared as Preparation Example 1 was administered, and as a result, it was found that body weight recovery more quickly occurred in all test groups compared to the CTX administration group.

Next, CBC measurement was performed as follows. Specifically, all mice were anesthetized with 1% to 2% isoflurane before sacrifice, and whole blood was collected from the abdominal aortic vein. Afterwards, blood was collected using EDTA lavender tubes (Becton, Dickinson and Company, Franklin Lakes, NJ, USA), and the collected blood was subjected to CBC (Complete Blood Count) analysis using the ADVIA 2120i hematology system (Siemens, Munich, Germany).

At this time, an increase in WBC indicates immune function activation.

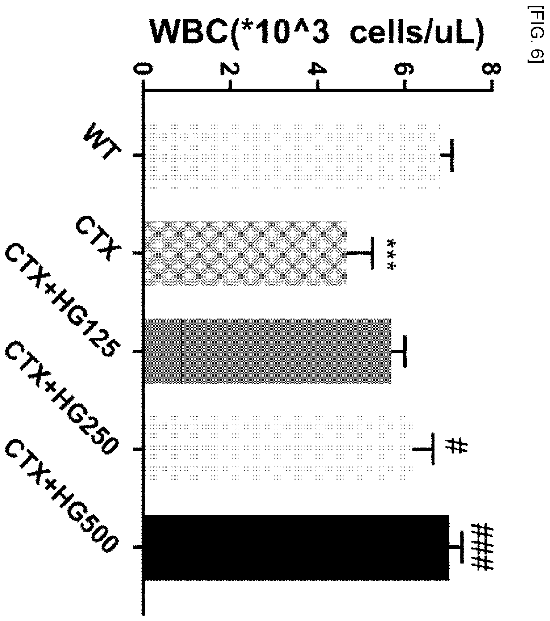

As a result, as can be seen from FIG. 6, it was found that WBC was significantly reduced in the CTX administration group, indicating that immunosuppression was well achieved. Afterwards, it was found that the WBC count significantly increased in the group treated with HG of Preparation Example 1 of the present invention, indicating that the immune function was restored compared to the CTX treatment group.

### 3-3. Examination of increase in NK cell activity in group administered with mixture prepared as Preparation Example 1

In order to examine the effect of the mixture prepared as Preparation Example 1 on innate immunity among the types of immunity in the group administered with the mixture, NK cell activity was examined.

The immunocompromised animals constructed in Example 3-1 were used, and NK cell activity was examined by the following method.

Before sacrificing mice, all mice were anesthetized using 1% to 2% isoflurane, blood was collected, and then 0.8 mL of blood was transferred to a tube containing heparin (JW Pharmaceutical, Seocho, Korea). After treatment with an activator, the blood was placed in a tube and gently mixed at room temperature for less than 1 hour. Then, the supernatant was placed in a 96-well plate and incubated at 37°C for 24 hours. After incubation, samples were placed in the stained plates, and IFN-γ expression was analyzed using the Murine NK Activation Kit (NK MAX Bio, Seongnam, Korea).

Here, NK cells are known to function in the spleen and bone marrow and regulate innate immunity in the body, and NK cell activity is measured by IFN-γ expression.

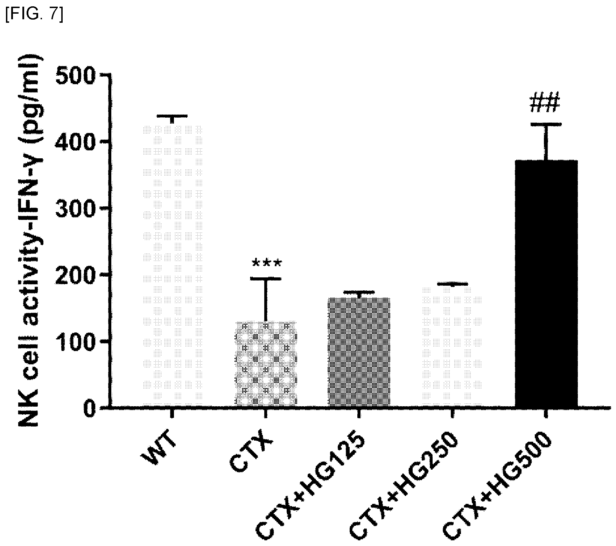

As a result, as can be seen from FIG. 7, it was found that IFN-γ expression decreased rapidly in the CTX administration group, resulting in immunosuppression. In contrast, it was found that IFN-γ expression significantly increased by about 3 times or more in the group treated with HG of Preparation Example 1, which is a mixture of the present invention.

Through this, it can be seen that NK cell activity significantly increases in the group treated with HG of the present invention and this significantly increases innate immunity.

### 3-4. Examination of recovery of spleen cell density in group administered with mixture prepared as Preparation Example 1

It was examined whether the spleen cell density was restored in the group administered with the mixture prepared as Preparation Example 1.

The immunocompromised animals constructed in Example 3-1 were used, and hematoxylin and eosin staining of the spleen was performed according to the following process. Specifically, spleen tissue isolated from mice was fixed with 10% neutral buffered formalin (Biosesang, Yongin, Korea), embedded in paraffin, and then sectioned into a size of 3 µm using the Finesse ME microtome (Thermo Fisher Scientific, Massachusetts, USA). Then, the sections were dried, paraffin was removed, and then the sections were washed with distilled water and stained with a hematoxylin and eosin solution.

Afterwards, the stained slides were scanned under a microscope and the white pulp appearance was compared and analyzed.

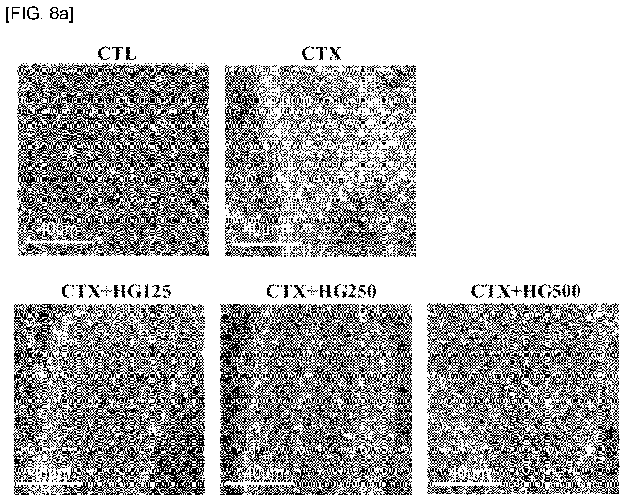

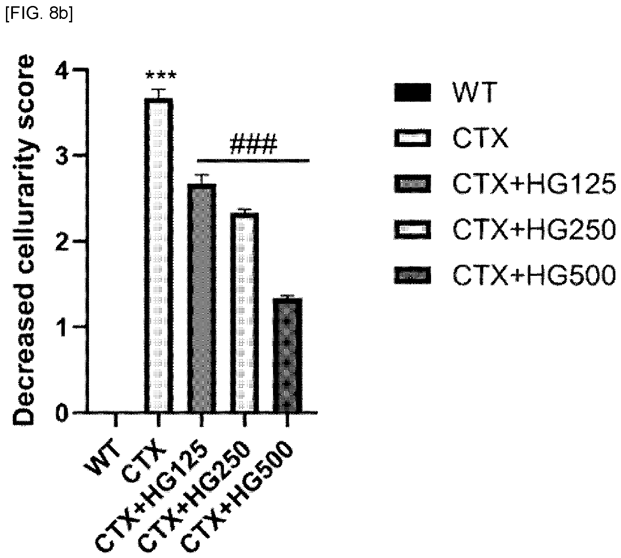

At this time, the cell density of white pulp is an important factor in evaluating immunity enhancement, and the results are shown in FIGS. 8A and 8B.

As can be seen from the figures, a number of dead cells are visible in the CTX administration group and the cell density is reduced. On the other hand, in the group administered with HG of Preparation Example 1, which is a mixture of the present invention, it can be seen that the reduced cell density is restored.

### 3-5. Examination of increase in cytokine expression in group administered with mixture prepared as Preparation Example 1

The spleen cell proliferation ability in the group administered with the mixture prepared as Preparation Example 1 was examined.

As immunocompromised animals, the animal model constructed in Example 3-1 was used for examination, and spleen cytokines were analyzed as follows. At this time, IL-2, IL-4, IFN-γ, and TNF-α as cytokines were analyzed.

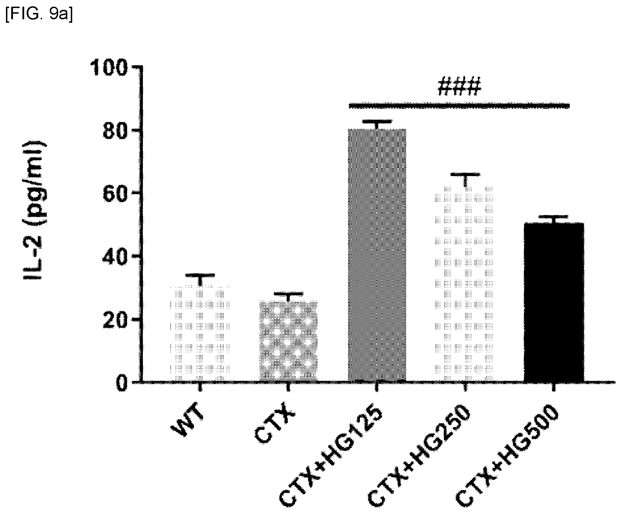

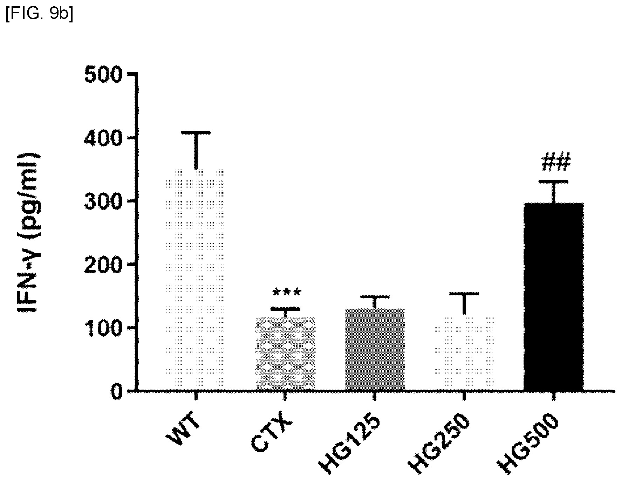

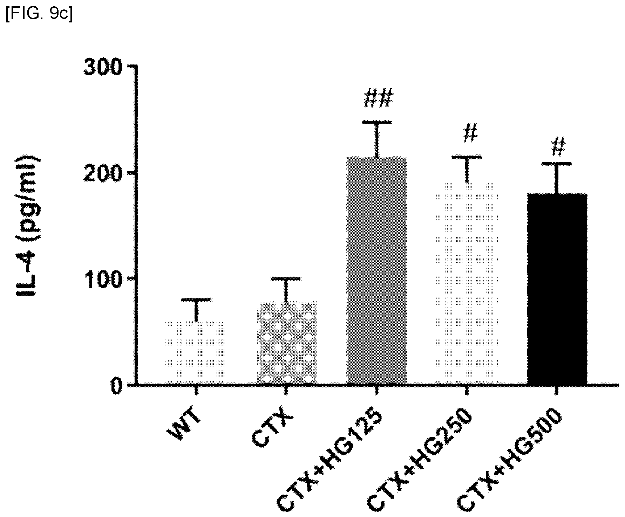

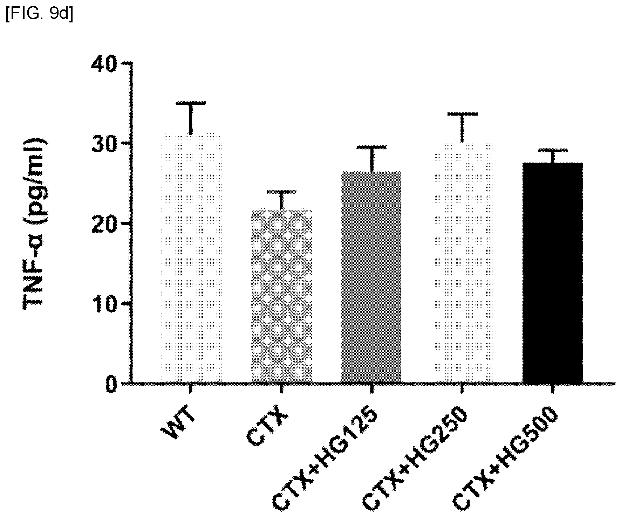

Specifically, spleen cells isolated from spleen tissue of experimental mice were aseptically treated and meshed in RPMI 1640 medium (Gibco, Grand Island, USA) containing 10% FBS (Gibco, Grand Island, USA) and 1% antibiotic and antimycotic (Gibco, Grand Island, USA). Afterward, the cell number per well was calculated to be 1 × 10⁶ cells/ml and the cells were dispensed into a 48-well plate so that the total volume was 150 µL. Then, the cells were treated with 50 ng/ml phorbol 12-myristate 13-acetate (MERCK, Darmstadt, Germany) and 2 µg/ml ionomycin (MERCK, Darmstadt, Germany) diluted with RPMI 1640 medium, and incubated at 37°C for 48 hours and 5% CO₂ conditions, then the supernatant was used for ELISA analysis (R&D Systems, Minneapolis, USA), and the absorbance at 450 nm was measured. At this time, the cytokine expression level due to the treatment with a sample was compared, and the results are shown in FIG. 9.

Here, cytokines are produced by activated lymphocytes, and an increase in cytokines indicates immune function enhancement.

As a result, as can be seen from FIG. 9, it has been found that the cytokines in the CTX-induced model increased equally in the test group, showing a correlation.

In particular, in the group of HG of Preparation Example 1 of the present invention, it can be seen that the expression of all cytokines, namely IL-2, IFN-γ, TNF-α, and IL-4, is significantly increased.

### 3-6. Examination of spleen cell proliferation ability in group administered with mixture prepared as Preparation Example 1

The spleen cell proliferation ability in the group administered with the mixture prepared as Preparation Example 1 was examined.

For this experiment, a normal model, not the immunocompromised animal constructed in Example 3-1, was used for examination, and the spleen cell proliferation ability was analyzed as follows.

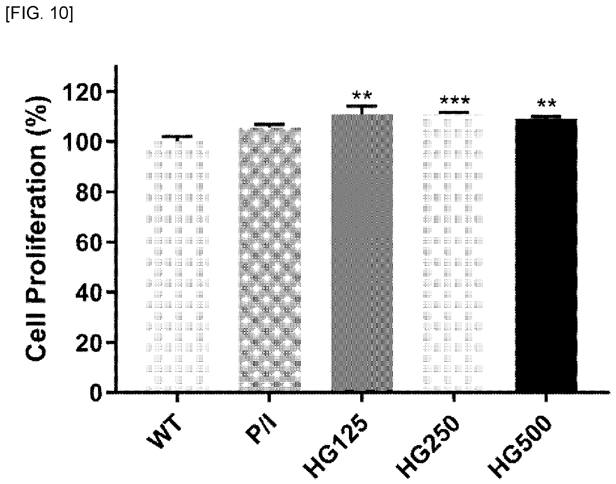

Specifically, spleen cells were isolated from spleen tissue of experimental mice, and then the spleen was aseptically treated and meshed in RPMI 1640 medium (Gibco, Grand Island, USA) containing 10% FBS (Gibco, Grand Island, USA) and 1% antibiotic and antimycotic (Gibco, Grand Island, USA). Next, the cell number per well was calculated to be 1 × 10⁶ cells/ml and the cells were dispensed into a 96-well plate so that the total volume was 100 µL. The cells were treated with 50 ng/ml phorbol 12-myristate 13-acetate (MERCK, Darmstadt, Germany), 2 µg/ml ionomycin (MERCK, Darmstadt, Germany) and HG diluted with RPMI 1640 medium according to concentration, and incubated for 35 hours under 37°C and 5% CO₂ conditions, then treated with WST-8 for 1 hour, and reacted in a dark state. After the reaction was completed, the OD value as the absorbance at 450 nm was checked using a microplate reader (TECAN, Mannedorf, Swiss) and is shown in FIG. 10.

The spleen is a maturation organ for immune cells, and this means that the immune function is improved as the spleen cell proliferation ability increases.

As a result, as can be seen from FIG. 10, it has been found that spleen cells significantly proliferate in all test groups of HG of Preparation Example 1, which is a mixture of the present invention.

In summary, it has been found that a mixture in which a mixed extract of angelica, cnidium, and paeonia and an ethanol polysaccharide, which is a polysaccharide isolated therefrom, is mixed in the present invention increases immunity, in particular, when a mixed extract in which *Angelica sinensis* (Oliv.) Diels, *Ligusticum chuanxiong* Hort. or *Cnidium officinale,* and *Paeonia lactiflora* Pallas are mixed at a weight ratio of 1 : 1.6 : 1.3 and a polysaccharide isolated therefrom are used in combination, immunity is increased more effectively, the mixture remarkably increases NK cell activity among others and thus exhibits a greatly excellent effect of enhancing innate immunity, and further, a water fraction and an ethanol fraction of the mixture are greatly excellent in enhancing immunity.

### 3-7. Statistical analysis

All of the data were analyzed using PRISM 7 (Graphpad Software, California, USA). Continuous data were expressed as mean ± S.E.M. For all groups, consistent variables were compared using Dunnet's multiple comparison test and one-way ANOVA. A p-value of <0.05 is considered statistically significant.

Consequently, it is possible to utilize the mixture in which a mixed extract of angelica, cnidium, and paeonia and an ethanol polysaccharide of the mixed extract are mixed of the present invention, or a fraction of the mixture as an enhancing agent of immunity including innate immunity.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within the scope of the claims or equivalents of such scope are therefore intended to be embraced by the claims.

## Claims

1. A composition for enhancing immunity, the composition comprising a mixture of a mixed extract of angelica, cnidium, and paeonia and a polysaccharide isolated from the mixed extract or a fraction of the mixture as an effective ingredient.

2. The composition according to claim 1, wherein the immunity is innate immunity, acquired immunity, or a combination thereof.

3. The composition according to claim 1, wherein the mixed extract is extracted with a solvent selected from the group consisting of water, a linear or branched alcohol having 1 to 6 carbon atoms, an organic solvent, and a mixed solvent of these solvents.

4. The composition according to claim 1, wherein the mixed extract contains angelica, cnidium, and paeonia at a weight ratio of 1 : 1.2 to 3 : 1.2 to 3.

5. The composition according to claim 1, wherein the mixed extract is a mixed extract of *Angelica sinensis* (Oliv.) Diels, *Ligusticum chuanxiong* Hort. or *Cnidium officinale*, and *Paeonia lactiflora* Pallas.

6. The composition according to claim 1, wherein the isolated polysaccharide is an ethanol polysaccharide.

7. The composition according to claim 6, wherein the ethanol is 95% ethanol.

8. The composition according to claim 1, wherein the fraction is a water or ethanol fraction of the mixture.

9. The composition according to claim 8, wherein the water fraction or ethanol fraction has T cell proliferation ability.

10. The composition according to claim 8, wherein the water fraction or ethanol fraction has a T cell proliferation ability of 130% to 160% at a concentration of 400 to 500 µg/ml.

11. The composition according to claim 1, wherein the mixture contains a mixed extract of angelica, cnidium, and paeonia and a polysaccharide isolated from the mixed extract at a ratio of 60 to 70 : 30 to 40.

12. The composition according to claim 1, wherein the mixture is contained at 0.001% to 90% by weight based on the total weight of the composition.

13. The composition according to claim 1, wherein the mixture or a fraction of the mixture increases NK cell activity.

14. The composition according to claim 1, wherein the mixture or a fraction of the mixture increases body weight, increases WBC count, restores spleen cell density, increases spleen cell proliferation ability, and increases immune-related cytokines.

15. The composition according to claim 14, wherein the cytokine is any one or more selected from the group consisting of IL-2, IFN-γ, IL-4, IL-10, TNF-α, IL-12, and IL-6.

16. The composition according to claim 1, which is a food composition.

17. The composition according to claim 16, wherein the food composition is prepared as a powder, granule, tablet, capsule, syrup, or beverage.
